# EUROPÄISCHE PATENTSCHRIFT

(11) **EP 1 856 277 B1**
(45) Veröffentlichungstag und Bekanntmachung des Hinweises auf die Patenterteilung: **22.06.2011**
(21) Anmeldenummer: 06707345.2
(22) Anmeldetag: 01.03.2006
(51) Int. Cl.: C12Q 1/68

(54) **MARKIERUNGSLÖSUNG ZUM AUFTRAGEN MITTELS EINES TINTENSTRAHLDRUCKERS**
MARKER SOLUTION TO BE APPLIED BY MEANS OF AN INKJET PRINTER
SOLUTION DE MARQUAGE A APPLIQUER AU MOYEN D'UNE IMPRIMANTE A JET D'ENCRE

(30) Priorität: 04.03.2005 DE 102005009943; 18.03.2005 DE 102005012567
(43) Veröffentlichungstag der Anmeldung: 21.11.2007
(73) Patentinhaber: Secutech International Pte. Ltd., Singapore 069443 (SG)
(72) Erfinder: JOSTEN, Andre, 90429 Nürnberg (DE)
(74) Vertreter: UEXKÜLL & STOLBERG
(86) Internationale Anmeldenummer: PCT/EP2006/001863
(87) Internationale Veröffentlichungsnummer: WO 2006/092286

(56) Entgegenhaltungen:
- EP-A- 0 272 896
- WO-A-02/072878
- WO-A-03/038000
- WO-A-2004/053161
- US-A1- 2004 054 160

### Sequenzprotokoll

<110> identif GmbH
<120> Markierungslösung zum Auftragen mittels eines Tintenstrahldruckers
<130> 464363EH
<160> 2
<170> PatentIn version 3.1
<210> 1
   <211> 25
   <212> DNA
   <213> Künstliche Sequenz
<220>
   <223> Oligonukleotid
<400> 1
   tggagggatg atactttgcg cttgg 25
<210> 2
   <211> 32
   <212> DNA
   <213> Künstliche Sequenz
<220>
   <223> 5'-6FAM- und 3'dt(C2-DABCYL)-markiertes Oligonukleotid
<400> 2
   ccaagcgcaa agtatcatcc ctccaggctt gg 32

## Beschreibung

Die Erfindung betrifft eine Markierungslösung, die zur Herstellung fälschungssichere Markierungen geeignet ist und mit handels- und industrie-üblichen Druckern, z. B. Tintenstrahldruckern, aufgebracht werden kann. Insbesondere betrifft die Erfindung eine Markierungslösung, die mindestens ein organisches Lösungsmittel mit einem bei 20 °C höheren Dampfdruck als Wasser enthält. Derartige Markierungslösungen können in Form von Tinten bei, insbesondere industriell eingesetzten Tintenstrahldruckern verwendet werden. Sie weisen den Vorteil auf, schnell zu trocknen und auch auf hydrophoben Oberflächen eine genau definierte Markierung zu ermöglichen. Die Erfindung betrifft weiterhin ein Verfahren zur Herstellung der Markierungslösung und eine Verwendung der Markierungslösung zum Markieren von Objekten.

Aus der US 2003/0229222 A1 ist ein Verfahren zum Extrahieren von Nukleinsäure aus biologischem Material bekannt. Bei dem Verfahren wird das biologische Material mit kleinen Partikeln sowie mit einem Zwei-Phasen-Gemisch aus einer Komplexbildner und quaternäre Ammoniumsalze enthaltenden wässrigen Lösung und einem organischen Lösungsmittel kräftig geschüttelt. Nach der Extraktion wird aus der wässrigen Phase durch Ethanolpräzipitation die Nukleinsäure gewonnen. Das organische Lösungsmittel ermöglicht eine effiziente Reinigung, weil in dem biologischen Material enthaltene Kontaminationen leicht in das organische Lösungsmittel aufgenommen werden.

Aus der WO 03/038000 A1 ist eine Markierungslösung zur fälschungssicheren Kennzeichnung eines Wertgegenstands bekannt. Die Markierungslösung umfasst eine wässrige Lösung mit einzelsträngigen Nukleinsäuren sowie Glyzerin und Polyethylenglykol. Das Glyzerin wirkt dabei als hygroskopische Substanz und hält die Markierung stets feucht. Dadurch wird eine gute Hybridisierbarkeit mit einer zum Nachweis der Markierung zu verwendenden komplementären Nukleinsäure erreicht. Die Markierungslösung kann beispielsweise aufgestempelt werden. Für den Einsatz in einem Tintenstrahldrucker ist sie wegen der durch Glyzerin und Polyethylenglykol bedingten Viskosität und der dadurch ebenfalls bedingten langsamen und unvollständigen Trocknung ungeeignet.

Aus der US 2004/0054160 A1 ist ein Medium oder eine Tintenlösung bekannt, welche Nukleinsäure enthält. Das Medium enthält zwischen 30 und 80 Volumenprozent einer organischen Lösung umfassend Dimethylsulfoxid, Ethylenglykol, Formamid oder eine Kombination daraus. Das Medium ist ebenfalls zum Stempeln geeignet. Das Medium verdampft durch einen reduzierten Wasseranteil langsamer als herkömmliche Tinte. Dadurch erfolgt die durch Verdunstung bedingte Veränderung der Zusammensetzung der Tinte langsamer, so dass konstantere Druckergebnisse erzielt werden. Ein schnelltrocknendes Medium wird durch die US 2004/0054160 A1 nicht offenbart.

Aus der EP 0 895 082 B1 ist ein "Bubble-jet"-Verfahren zur Probenaufgabe auf festen Trägern bekannt. Die aufgetragene Flüssigkeit kann eine Nukleinsäure und 5 bis 10 Gew.% Harnstoff, 5 bis 10 Gew.% Glyzerin, 5 bis 10 Gew.% Thiodiglykol und 1% eines Acetylenalkohols enthalten. Auch diese Flüssigkeit ist nicht schnelltrocknend. Das Glyzerin bewirkt, dass eine aufgetragene Probe dauerhaft feucht bleibt.

Bei industriell eingesetzten Tintenstrahldruckern ist es bekannt, eine schnelltrocknende Tinte einzusetzen, die ein organisches Lösungsmittel mit einem bei 20 °C höheren Dampfdruck als Wasser aufweist. Tinten auf Wasserbasis ohne organische Lösungsmittel sind auf Grund der langsamen Trocknung nur zur Auftragung auf saugfähige Materialien oder für Kennzeichnungsprozesse, bei denen eine ausreichende lange Trocknungszeit zur Verfügung steht, geeignet. Für schnelle Kennzeichnungsprozesse oder zur Auftragung auf hydrophoben und/oder nicht saugfähigen Oberflächen, wie beispielsweise Hochglanzoberflächen oder, insbesondere glatten, Kunststoffoberflächen, sind auf organischen Lösungsmitteln basierende Tinten erforderlich. Herkömmliche Nukleinsäuren sind in solchen Tinten nicht oder nur in niedriger Konzentration zu lösen. Die Menge der mit einer solchen Tinte auftragbaren Nukleinsäure ist zu gering, um sie ohne Vervielfältigungsreaktion nachweisen zu können.

Aufgabe der vorliegenden Erfindung ist es, eine zur Auftragung mittels eines Druckers geeignete schnelltrocknende nukleinsäurehaltige Markierungslösung bereitzustellen. Insbesondere soll mittels der Markierungslösung Nukleinsäure z. B. mittels eines Tintenstrahldruckers in einer solchen Menge auftragbar sein, dass ein direkter Nachweis ohne die Durchführung einer Vervielfältigungsreaktion auf der markierten Oberfläche möglich ist. Ein weiteres Ziel der Erfindung ist es, eine Nukleinsäure-haltige Markierungsflüssigkeit bereitzustellen, deren Nukleinsäuren im verdrucken Zustand direkt einer Hybridisierung mit komplementären Nukleinsäuren zugänglich sind.

Die Aufgabe wird durch die Merkmale der Ansprüche 1, 21 und 40 gelöst. Zweckmäßige Ausgestaltungen ergeben sich aus den Merkmalen der Ansprüche 2 bis 20, 22 bis 39 und 41 bis 46.

Erfindungsgemäß ist eine Markierungslösung zum Auftragen mittels eines Tintenstrahldruckers vorgesehen, die als Komponenten (i) mindestens ein organisches Lösungsmittel mit einem bei 20°C höheren Dampfdruck als Wasser und einem Wassergehalt von weniger als 50 % (v/v), (ii) vorgegebene erste synthetisch hergestellte Nukleinsäuren und (iii) einen die ersten Nukleinsäuren komplexierenden, organischen Hilfsstoff enthält.

Die Erfinder der vorliegenden Erfindung haben erkannt, dass es durch das Vorsehen des die ersten Nukleinsäuren komplexierenden Hilfsstoff möglich ist, erste Nukleinsäuren, insbesondere DNA, auch in einer auf einem organischen Lösungsmittel basierenden Markierungslösung in verhältnismäßig hoher Konzentration zu lösen. Es ist dadurch möglich, mittels der Markierungslösung und z. B. einem Tintenstrahldrucker DNA in einer solchen Menge auf einer Oberfläche aufzutragen, dass diese direkt, d.h. ohne eine vorhergehende Amplifikation, mittels eines Vor-Ort-Tests nachgewiesen werden kann. Es hat sich weiterhin gezeigt, dass die mit dem Hilfsstoff komplexierten Nukleinsäuren erstaunlicherweise nach dem Trocknen auf der Oberfläche trotz der Trocknung und trotz des Vorhandenseins des Hilfsstoffs in einer solchen Art vorliegen, dass sie direkt einer Hybridisierung mit einer in wässriger Lösung vorliegenden komplementären Nukleinsäure zugänglich sind. Der Nachweis einer mittels der erfindungsgemäßen Markierungslösung hergestellten Markierung kann somit schnell und einfach direkt auf der markierten Oberfläche durchgeführt werden. Es ist für den Nachweis nicht erforderlich, die Nukleinsäuren von der Oberfläche zu entfernen. Der Nachweis der so vorliegenden ersten Nukleinsäuren kann beispielsweise mittels eines Verfahrens erfolgen, wie es aus der WO 01/51652 A2 bekannt ist. Die erste Nukleinsäure ist vorzugsweise eine synthetisch hergestellte Nukleinsäure. Dadurch kann gut eine genau definierte Codierung bereitgestellt werden.

Es ist möglich, die Markierung durch einen Kontakt mit gelösten komplementären Nachweisnukleinsäuren am Ort der Markierung direkt auf der Oberfläche nachzuweisen. Der Nachweis der Hybridisierung kann z.B. anhand von veränderten optischen Eigenschaften der Nachweisnukleinsäuren beobachtet werden. Als Nachweisnukleinsäuren können dabei z.B. Molecular Beacons verwendet werden, die mittels eines Stifts oder einer Dosiereinrichtung auf die Markierung aufgebracht werden. Der Nachweis der durch die Hybridisierung veränderten Fluoreszenz der Molecular Beacons kann mittels eines Handscanners erfolgen. Durch diese Eigenschaften ist eine Identifizierung der Markierung vor Ort, d.h. ohne Laboreinrichtung, auch von ungeschultem Personal leicht durchführbar.

Die erfindungsgemäße Markierungslösung hat den weiteren Vorteil, dass sie keine Partikel enthält, welche eine Düse eines Druckkopfes eines Tintenstrahldruckers verstopfen können. Ein weiterer Vorteil der erfindungsgemäßen Markierungslösung besteht darin, dass in der Markierungslösung eine so große Menge an Nukleinsäure gelöst werden kann, dass neben der spezifischen, der Markierung dienenden ersten Nukleinsäure eine große Menge unspezifischer Nukleinsäure gelöst werden kann. Dadurch kann die spezifische Nukleinsäure sozusagen in der unspezifischen Nukleinsäure versteckt werden. Es ist dann schwer möglich, die spezifische Nukleinsäure zu analysieren und nachzumachen, um dadurch eine gefälschte Markierung aufbringen zu können. Die Markierungslösung kann dadurch sehr fälschungssicher gestaltet werden. Es wurde festgestellt, dass in der erfindungsgemäßen Markierungslösung 3 Gew.% Nukleinsäure gelöst sein kann. Ein weiterer Vorteil der erfindungsgemäßen Markierungslösung besteht darin, dass sie in getrocknetem Zustand nahezu unsichtbar ist. Dadurch ist die Stelle, an welcher die Markierung auf einem damit markierten Objekts angebracht ist, nicht ohne weiteres zu erkennen. Die Markierung kann nur schwer aufgefunden werden, um sie zu analysieren und ggf. nachzumachen. Mit der erfindungsgemäßen Markierungslösung sind dadurch sehr fälschungssichere Markierungen möglich.

Darüber hinaus haftet die mit der erfindungsgemäßen Markierungslösung aufgebrachte Markierung insbesondere auf glatten und sogar hydrophoben Oberflächen gut, ohne dass diese einer entsprechenden Vorbehandlung unterzogen werden müssten. Durch das organische Lösungsmittel wird darüber hinaus auch eine gute Benetzbarkeit hydrophober Oberflächen erreicht.

Da organische Lösungsmittel wie Isopropanol oder Ethanol in der Molekularbiologie üblicherweise zum Fällen von Nukleinsäure verwendet werden, ist es sehr erstaunlich, dass in diesen Lösungsmitteln mittels eines die ersten Nukleinsäuren komplexierenden Hilfsstoffs, wie z.B. eines kationischen Detergens, eine verhältnismäßig große Menge an DNA gelöst werden kann.

Zum Erhöhen der Löslichkeit in organischen Lösungsmitteln könnten die ersten Nukleinsäuren auch modifiziert werden. Beispielsweise könnten Methyl- oder Cholesteryl-Gruppen bei der Nukleinsäuresynthese oder durch nachträgliche Modifikation an den ersten Nukleinsäuren vorgesehen werden. Nachteilig an einer chemischen Modifikation der ersten Nukleinsäuren sind jedoch die erhöhten Kosten und die nicht erwünschte Möglichkeit die ersten Nukleinsäuren anhand der Modifikation zu isolieren. Das hier offenbarte Verfahren eröffnet die Möglichkeit erste Nukleinsäuren in hohen Konzentrationen in organischen Lösungsmitteln zu lösen, ohne auf eine Modifikation der ersten Nukleinsäuren angewiesen zu sein.

Besonderes vorteilhaft ist es, wenn die Markierungslösung insgesamt bei 20 °C einen höheren Dampfdruck als Wasser aufweist. Dadurch wird die Trocknung der aufgetragenen Markierungslösung weiter beschleunigt. Der Dampfdruck des organischen Lösungsmittels oder der Markierungslösung bei 20 °C beträgt vorzugsweise mehr als 0,025 bar, insbesondere mehr als 0,027 bar, vorzugsweise mehr als 0,03 bar. Das organische Lösungsmittel kann Methanol, Ethanol, Propanol, Isopropylalkohol, Butanon, Aceton, Ethylether, Benzol oder eine Mischung von mindestens zwei dieser Lösungsmittel sein.

Vorzugsweise ist die Konzentration des organischen Lösungsmittels in der Markierungslösung größer als 50 % (v/v), bevorzugt größer als 80 % (v/v), insbesondere größer als 90% (v/v). (v/v) bedeutet (Volumen/Volumen). Je höher der Anteil des organischen Lösungsmittels in der Markierungslösung ist, desto schneller trocknend ist die Markierungslösung. Die Konzentration von Wasser in der Markierungslösung ist bevorzugt kleiner als 20 % (v/v), besonders bevorzugt kleiner als 10 % (v/v), insbesondere kleiner als 5 % (v/v).

In einer Ausgestaltung der Erfindung enthält die Markierungslösung weiterhin einen, insbesondere fluoreszierenden - Farbstoff. Dadurch kann die Markierung zur Identifizierung leichter lokalisiert werden.

Besonders günstig ist es, wenn die ersten Nukleinsäuren in der Markierungslösung einzelsträngig vorliegen. Dann ist zum direkten Nachweis der ersten Nukleinsäuren kein Denaturierungsschritt erforderlich. Es hat sich erstaunlicherweise gezeigt, dass die ersten Nukleinsäuren in der getrockneten, aufgedruckten Markierung direkt einer Hybridisierung zugänglich ist. Dies ist umso erstaunlicher, da der Nachweis durch Hybridisierung in Gegenwart, des in der Markierung befindlichen, die ersten Nukleinsäuren komplexierenden Hilfsstoffs, wie z.B. eines kationischen Detergens, erfolgt. Der Nachweis einer mittels der Markierungslösung hergestellten Markierung kann somit schnell und einfach auf der markierten Oberfläche durchgeführt werden.

Der Nachweis in der Markierung enthaltenen ersten Nukleinsäuren kann durch ein Zufügen einer geringen Menge von z.B. 1 bis 10µl einer Identifizierungslösung erfolgen, wobei die Identifizierungslösung Nachweisnukleinsäuren enthält, die zu den ersten Nukleinsäuren komplementär sind. Das Zufügen kann z.B. mittels eines Stifts erfolgen, der mit der Markierung in Kontakt gebracht wird und mittels Kapillarkräfte eine geringe Menge der Identifizierungslösung in die Markierung abgibt. Selbstverständlich kann die Identifizierungslösung auch mittels einer Pipetiervorrichtung auf die Markierung aufgebracht werden. Der Nachweis der Hybridisierung erfolgt vorzugsweise mittels komplementärer Molecular Beacon anhand der durch die Hybridisierung veränderte Fluoreszenz. Die veränderte Fluoreszenz kann z.B. mittels eines Hand-Fluoreszenz-Scanners durchgeführt werden. In dieser Ausgestaltung ist ein Vorort-Nachweis der Markierung ohne Laborausstattung und ohne wissenschaftlich geschultes Personal möglich.

Zum Einstellen der Viskosität der Markierungslösung können darin visköse Polymere enthalten sein. Das Einstellen der Viskosität kann erforderlich sein, um die Markierungslösung an die Erfordernisse verschiedener Druckköpfe und der darin vorhandenen Düsen anzupassen. Zur Einstellung der Leitfähigkeit der Markierungslösung können darin geladene Moleküle, insbesondere Tetrabutylammoniumbromid, enthalten sein. Das Einstellen der Leitfähigkeit kann bei Tintenstrahldruckern erforderlich sein, bei denen der Auftragungsort der Tinte durch Ablenkung eines Tintenstrahls im elektrischen Feld erfolgt. Es muss eine bestimmte Leitfähigkeit der Markierungslösung eingestellt werden, damit der Tintenstrahl bei gegebener Feldstärke die gewünschte Ablenkung erfährt.

Die ersten Nukleinsäuren können eine Länge von 5 bis 300 Nukleotiden, insbesondere 10 bis 100 Nukleotiden, vorzugsweise 15 bis 25 Nukleotiden, aufweisen. Je größer die Länge der Nukleinsäuren ist, desto größer ist die Menge der dadurch bereitstellbaren Codierungen. Gleichzeitig wird es mit zunehmender Länge aber aufwändiger, fehlerfreie definierte Nukleinsäuren bereitzustellen. Die angegebenen Längen haben sich als günstig erwiesen.

Die Sicherheit einer mittels der Markierungslösung bereitgestellten Markierung kann weiter erhöht werden, wenn darin, insbesondere eine ähnliche Länge wie die erste Nukleinsäuren aufweisende, weitere Nukleinsäuren enthalten sind. Durch die weiteren Nukleinsäuren ist es möglich, die ersten Nukleinsäuren innerhalb einer Menge weiterer Nukleinsäuren zu verstekken. Dadurch ist es nicht möglich, die Sequenz der ersten Nukleinsäure zu ermitteln und damit die Markierungslösung bzw. die damit aufgebrachte Markierung zu fälschen.

Das Mengenverhältnis zwischen den ersten und den weiteren Nukleinsäuren beträgt vorzugsweise höchstens 1:10, besonders bevorzugt höchstens 1:20, insbesondere höchstens 1:100. Je kleiner das Mengenverhältnis ist, desto fälschungssicherer ist die mittels der Markierungslösung hergestellte Markierung, weil es dadurch immer schwieriger wird, die ersten Nukleinsäuren unter den insgesamt vorhandenen ersten und weiteren Nukleinsäuren zu isolieren und zu identifizieren.

Die Konzentration der ersten oder der ersten und der weiteren Nukleinsäuren in der Markierungslösung ist vorzugsweise höher als 0,1 % (w/v), insbesondere höher als 1 % (w/v), vorzugsweise höher als 2 % (w/v). Eine hohe Konzentration der ersten Nukleinsäuren in der Markierungslösung erleichtert einen amplifikations-freien Nachweis der ersten Nukleinsäure in der gedruckten Markierung.

Unter dem ersten Nukleinsäuren komplexierenden Hilfsstoff wird im Rahmen der vorliegenden Erfindung eine organische, die ersten Nukleinsäuren bindende Verbindung angesehen, die zumindest eine positive Ladung oder positive Partialladung trägt und über organische Reste verfügt. Der Hilfsstoff kann auch ein Polymer sein, das pro Molekül mehrere positive Ladungen trägt. Typische Vertreter der Hilfsstoffe sind kationischen Detergentien, wie z.B. quartäre Ammoniumverbindungen mit unterschiedlichen Alkylresten. Die Konzentration des Detergens in der Markierungslösung kann zwischen 0,1 % (w/v) und 10 % (w/v), insbesondere zwischen 0,5 % (w/v) und 5 % (w/v), vorzugsweise zwischen 0,7 % (w/v) und 3 % (w/v), betragen. (w/v) bedeutet (Gewicht/Volumen). Es können auch organische, amphiphile Verbindung, wie z.B. organische Amine als Hilfsstoffe zur Verwendung kommen. Bevorzugt können Hilfsstoffe wie Spermidin, Spermin oder Polylysin, die in wässrigen Lösungen mit Nukleinsäuren unlösliche Komplexe bilden, verwendet werden.

Bei einer bevorzugten Ausgestaltung der erfindungsgemäßen Markierungslösung wird als Hilfsstoff das kationische Detergens Hexadecyltrimethylammoniumbromid oder Dodecyltrimethylammoniumbromid verwendet. Vorzugsweise liegen die ersten und die weiteren Nukleinsäuren in der Markierungslösung in einer Konzentration gelöst vor, in welcher sie ohne den Hilfsstoff in der ansonsten unveränderten Markierungslösung nicht vollständig löslich wären. Die Konzentration der Nukleinsäuren in der Markierungslösung liegt bevorzugt um mehr als Faktor 10, bevorzugt Faktor 100 über der Konzentration, in welcher sie ohne den Hilfsstoff in der ansonsten unveränderten Markierungslösung löslich wären. Je höher die Konzentration der ersten und weiteren Nukleinsäuren in der Markierungslösung ist, desto größer ist die Menge dieser Nukleinsäuren, welche mittels eines Tintenstrahldruckers pro Flächeneinheit aufgetragen werden kann. Je größer die pro Flächeneinheit aufgetragene Menge ist, desto leichter ist der Nachweis dieser Nukleinsäuren und desto größer kann die Sicherheit der Markierung gestaltet werden, weil eine ohne weiteres nachweisbare Menge erster Nukleinsäuren in einer großen Menge weiterer Nukleinsäuren versteckt werden kann.

Die erfindungsgemäße Markierungslösung kann in einem Druckkopfs eines Tintenstrahldruckers in einem Vorratsbehälter oder in einem Kanal enthalten sein.

Weiterhin betrifft die Erfindung ein Verfahren zur Herstellung der erfindungsgemäßen Markierungslösung mit folgenden Schritten:
a) Lösen erster synthetisch hergestellter Nukleinsäuren in einem wässrigen Lösungsmittel oder Bereitstellen erster synthetisch hergestellter Nukleinsäuren in einem wässrigen Lösungsmittel,
b) In-Kontakt-Bringen der ersten Nukleinsäuren mit einem die ersten Nukleinsäuren komplexierenden, organischen Hilfsstoff, wobei sich Komplexe aus den ersten Nukleinsäuren und dem Hilfsstoff bilden,
c) Mischen der Komplexe mit einem organischen Lösungsmittel, das bei 20 °C einen höheren Dampfdruck als Wasser aufweist.

Bevorzugt findet das In-Kontakt-Bringen der ersten Nukleinsäuren mit dem Hilfsstoff in Schritt b) dadurch statt, dass eine Lösung mit dem Hilfsstoff vorgelegt wird, in die eine wässrige, die ersten Nukleinsäuren enthaltende Lösung eingebracht wird. Dadurch kann gewährleistet werden, dass der Hilfsstoff bei dem Kontakt mit den ersten Nukleinsäuren im Überschuss vorhanden ist und sich mit Hilfsstoff abgesättigte Nukleinsäurekomplexe bilden können.

Das Mischen der Komplexe mit dem organischen Lösungsmittel kann auch dadurch erfolgen, dass das die ersten Nukleinsäuren und den Hilfsstoff enthaltende, wässrige Lösungsmittel mit dem organischen Lösungsmittel gemischt wird.

Bevorzugt werden Hilfsstoff und das wässrige Lösungsmittel so gewählt, dass beim Schritt lit. b die Komplexe aus ersten Nukleinsäuren und Hilfsstoff ausfallen. Die ausgefallenen Komplexe können von dem wässrigen Lösungsmittel abgesondert und bei Schritt lit. c in dem organischen Lösungsmittel gelöst werden. Das Absondern der ausgefallenen Komplexe kann beispielsweise durch Zentrifugation oder durch Filtration erfolgen. Durch die Ausfällung und Abtrennung der Komplexe kann eine Konzentrierung der Nukleinsäuren erreicht werden.

Darüber hinaus betrifft die Erfindung eine Verwendung einer Markierungslösung, die als Komponenten (i) mindestens ein organisches Lösungsmittel mit einem bei 20 °C höheren Dampfdruck als Wasser und einem Wassergehalt von weniger als 50% (v/v), (ii) vorgegebene erste, insbesondere synthetisch hergestellte, Nukleinsäuren und (iii) einen die ersten Nukleinsäuren komplexierenden, organischen Hilfsstoff enthält, zum Markieren einer Oberfläche eines Objekts mittels eines Drukkers, insbesondere eines Tintenstrahldruckers.

Vorzugsweise ist die Oberfläche glatt und/oder besteht aus einem, insbesondere hydrophoben, Kunststoff. Bei dem Kunststoff kann es sich um Polyethylen oder Polypropylen handeln. Um die Fälschungssicherheit zu erhöhen, kann eine Markierung dadurch gebildet werden, dass die Markierungslösung auf definierte Flächen und eine andere Lösung auf weitere Flächen aufgebracht wird. Die andere Lösung ist dabei wie die Markierungslösung zusammengesetzt, ohne jedoch erste Nukleinsäuren zu enthalten. Dadurch ist es für einen Fälscher schwer herauszufinden, welche der Flächen die ersten Nukleinsäuren enthalten. Weiterhin kann die weitere Fläche beim Nachweis der Markierung als Referenzfläche dienen.

Weiter gesteigert werden kann die Sicherheit dadurch, dass die andere Lösung zweite Nukleinsäuren enthält, weil ein Fälscher dann zusätzlich herausfinden muss, ob die ersten oder die zweiten Nukleinsäuren die spezifische Codierung bereitstellen.

Zur Identifizierung einer mittels der Markierungslösung auf einer Oberfläche eines Objekts aufgebrachten Markierung kann eine spezifische Hybridisierung der ersten Nukleinsäuren mit dazu komplementären, d. h. spezifischen, dritten Nukleinsäuren erfolgen. Die dritten Nukleinsäuren können so beschaffen sein, dass sich durch die Hybridisierung Eigenschaften der dritten Nukleinsäure ändern. Diese sich ändernden Eigenschaften können zu einem auslesbaren Signal führen. Dazu können die dritten Nukleinsäuren beispielsweise *Molecular Beacons* sein. Das auslesbare Signal kann beispielsweise eine Farbänderung oder eine Fluoreszenzänderung sein. Die Sequenzen und die Sequenzlängen der zueinander komplementären Bereiche der ersten Nukleinsäuren und der dritten Nukleinsäuren werden vorzugsweise so gewählt, dass eine spezifische Hybridisierung bei Raumtemperatur möglich ist. Das erleichtert den spezifischen Nachweis der Markierung. Besonders bevorzugt ist es, wenn der Nachweis der Markierung in einer Einstufenreaktion, d. h. ohne Waschschritt, erfolgen kann. Ein derartiges Nachweisverfahren ist beispielsweise aus der WO 01/51652 A2 bekannt. Wenn die Markierung dadurch gebildet wird, dass die Markierungslösung auf definierte Flächen und die andere Lösung auf weitere Flächen aufgebracht wird, ist es beim Identifizieren der Markierung vorteilhaft, wenn auch die weiteren Flächen mit den dritten Nukleinsäuren in Kontakt gebracht werden, um zu sehen, dass dort keine spezifische Hybridisierung stattfindet. Findet dort doch eine spezifische Hybridisierung statt, spricht dies für eine gefälschte Markierung. Durch ein solches Vorgehen kann die Fälschungssicherheit des Verfahrens weiter erhöht werden.

Nachfolgend wird die Erfindung anhand eines Ausführungsbeispiels näher erläutert.

### 1) Herstellung von Markierungslösung

Zunächst wird eine Lösung aus 27,5 g DNA bestehend aus 2,5 g erster Nukleinsäure (Sequenz 1 = 5'-tg gagggatgat actttgcgct tgg-3') und 25 g Ultraschall-gescherter Heringssperm-DNA (Sigma, Best-Nr.D3159) in 500 ml Wasser hergestellt. Diese Lösung wird unter starkem Rühren zu 1500 ml einer 100 mmol/l enthaltenden Lösung von Dodecyltrimethylammoniumbromid (Sigma, Best-Nr.H9151) als Hilfsstoff in Wasser zugefügt. Es resultiert ein Niederschlag, bestehend aus DNA-Dodecyltrimethylammoniumbromid-Komplexen. Der Niederschlag wird durch Filtrieren abgetrennt und nach dem Trocknen bei Raumtemperatur in 1 1 Ethanol aufgenommen. Die so entstandene DNA-haltige ethanolische Lösung ist klar und kann selbst als Markierungslösung verwendet oder zur Herstellung anderer Markierungslösungen eingesetzt werden.

### 2) Herstellung der Markierung mittels Inkjet-Drucks

800 ml der Markierungslösung werden in einen Behälter überführt, der über eine Leitung mit dem Druckkopf einer Metronic Inkjet-Anlage (Veitshöchheim, Deutschland) verbunden ist. Mittels der Inkjet-Anlage werden auf einer am Druckkopf vorbeigeführten Kunststofffolie runde Markierungen mit einem Durchmesser von ca. 2 mm aufgebracht. In Abstand von ca. 5 mm von der ersten Markierung werden Kontrollmarkierung auf die Folie aufgebracht, die mit Markierungslösung ohne erste Nukleinsäuren hergestellt werden.

### 3) Nachweis der Markierung

Zur Identifizierung der Markierung wird nach zwei Wochen nach dem Aufdruck der Markierung mittels eines Stifts Detektionslösung auf die Markierung und die Kontrollmarkierung aufgebracht. Dazu wird der Stift ca. 2 Sekunden mit der Markierung in Kontakt gebracht. Die Detektionslösung besteht aus einer wässrigen Lösung die Molecular Beacon (Sequenz 2 = 5'-6FAM-ccaagcgcaa agtatcatcc ctccaggctt gg-Dabcyl-3') enthält, die partiell komplementär zu den ersten Nukleinsäuren in der Markierung sind. Unmittelbar nach dem Kontakt der Markierung und Kontrollmarkierung mit dem Stift wird mit einem Hand-Fluoreszenz-Scanner der Firma identif GmbH (Erlangen, Deutschland) die Fluoreszenz der Markierung und der Kontrollmarkierung bestimmt. Die Identifizierung der Markierung erfolgt anhand der erhöhten Fluoreszenz der Markierung gegenüber der Kontrollmarkierung.

## Patentansprüche

1. Markierungslösung zum Auftragen mittels eines Tintenstrahldruckers, die als Komponenten (i) mindestens ein organisches Lösungsmittel mit einem bei 20 °C höheren Dampfdruck als Wasser und einem Wassergehalt von weniger als 50% (v/v), (ii) vorgegebene erste synthetisch hergestellte Nukleinsäuren und (iii) einen die ersten Nukleinsäuren komplexierenden, organischen Hilfsstoff enthält.

2. Markierungslösung nach Anspruch 1, wobei die Markierungslösung bei 20 °C einen höheren Dampfdruck als Wasser aufweist.

3. Markierungslösung nach einem der vorhergehenden Ansprüche, wobei der Dampfdruck des organischen Lösungsmittels oder der Markierungslösung bei 20 °C mehr als 0,025 bar, insbesondere mehr als 0,027 bar, vorzugsweise mehr als 0,03 bar beträgt.

4. Markierungslösung nach einem der vorhergehenden Ansprüche, wobei das organische Lösungsmittel Methanol, Ethanol, Propanol, Isopropylalkohol, Butanon, Aceton, Ethylether, Benzol oder eine Mischung von mindestens zwei dieser Lösungsmittel ist.

5. Markierungslösung nach einem der vorhergehenden Ansprüche, wobei die Konzentration des organischen Lösungsmittels in der Markierungslösung größer als 50 % (v/v), bevorzugt größer als 80 % (v/v), insbesondere größer als 90 % (v/v) ist.

6. Markierungslösung nach einem der vorhergehenden Ansprüche, wobei die Konzentration von Wasser in der Markierungslösung kleiner als 20 % (v/v), bevorzugt kleiner als 10 % (v/v), insbesondere kleiner als 5 % (v/v) ist.

7. Markierungslösung nach einem der vorhergehenden Ansprüche, wobei die Markierungslösung weiterhin einen, insbesondere lumineszierenden, besonders bevorzugt fluoreszierenden, Farbstoff enthält.

8. Markierungslösung nach einem der vorhergehenden Ansprüche, wobei die ersten Nukleinsäuren einzelsträngig vorliegen.

9. Markierungslösung nach einem der vorhergehenden Ansprüche, wobei darin visköse Polymere zur Einstellung der Viskosität der Markierungslösung und/oder geladene Moleküle, insbesondere Tetrabutylammoniumbromid, zur Einstellung der Leitfähigkeit der Markierungslösung enthalten ist.

10. Markierungslösung nach einem der vorhergehenden Ansprüche, wobei die ersten Nukleinsäuren eine Länge von 5 bis 300 Nukleotiden, insbesondere 10 bis 100 Nukleotiden, vorzugsweise 15 bis 25 Nukleotiden, aufweisen.

11. Markierungslösung nach einem der vorhergehenden Ansprüche, wobei darin, insbesondere dieselbe oder nahezu dieselbe Länge wie die ersten Nukleinsäuren aufweisende, weitere Nukleinsäuren enthalten sind.

12. Markierungslösung nach Anspruch 11, wobei darin das Mengenverhältnis zwischen den ersten und den weiteren Nukleinsäuren höchstens 1:10, bevorzugt höchstens 1:20, insbesondere höchstens 1:100, beträgt.

13. Markierungslösung nach einem der vorhergehenden Ansprüche, wobei die Konzentration der ersten oder der ersten und der weiteren Nukleinsäuren in der Markierungslösung höher als 0,1 % (w/v), insbesondere höher als 1 % (w/v), vorzugsweise höher als 2 % (w/v), ist.

14. Markierungslösung nach einem der vorhergehenden Ansprüche, wobei der die ersten Nukleinsäuren komplexierende Hilfsstoff ein Detergens mit einer oder mehreren positiven Ladungen ist.

15. Markierungslösung nach Anspruch 14, wobei die Konzentration des Detergens in der Markierungslösung zwischen 0,1 % (w/v) und 10 % (w/v), insbesondere zwischen 0,5 % (w/v) und 5 % (w/v), vorzugsweise zwischen 0,7% (w/v) und 3 % (w/v), beiträgt.

16. Markierungslösung nach einem der vorhergehenden Ansprüche, wobei der die ersten Nukleinsäuren komplexierende Hilfsstoff ein monomeres oder polymeres Amin wie z.B. Spermidin oder Polylysin ist.

17. Markierungslösung nach einem der vorhergehenden Ansprüche, wobei der Hilfsstoff ein kationisches Detergens ist.

18. Markierungslösung nach Anspruch 17, wobei das kationische Detergens Hexadecyltrimethylammoniumbromid oder Dodecyltrimethylammoniumbromid ist.

19. Markierungslösung nach einem der vorhergehenden Ansprüche, wobei die ersten oder die weiteren Nukleinsäuren mit dem Hilfsstoff Komplexe bilden, die in wässriger Lösung unlöslich sind.

20. Markierungslösung nach einem der Ansprüche 11 bis 19, wobei die Löslichkeit der ersten und der weiteren Nukleinsäuren in der Markierungslösung um Faktor 5, bevorzugt Faktor 10, besonders bevorzugt Faktor 100 über der Löslichkeit in einer identischen Lösung ohne den Hilfsstoff ist.

21. Verfahren zur Herstellung der Markierungslösung nach einem der Ansprüche 1 bis 20, mit folgenden Schritten:
a) Lösen erster synthetisch hergestellter Nukleinsäuren in einem wässrigen Lösungsmittel oder Bereitstellen erster synthetisch hergestellter Nukleinsäuren in einem wässrigen Lösungsmittel,
b) In-Kontakt-Bringen der ersten Nukleinsäuren mit einem die ersten Nukleinsäuren komplexierenden, organischen Hilfsstoff, wobei sich Komplexe aus den ersten Nukleinsäuren und dem Hilfsstoff bilden,
c) Mischen der Komplexe mit einem organischen Lösungsmittel, das bei 20 °C einen höheren Dampfdruck als Wasser aufweist.

22. Verfahren nach Anspruch 21, wobei beim Schritt lit. b) ausfallende Komplexe von dem wässrigen Lösungsmittel abgesondert und bei Schritt lit. c) in dem organischen Lösungsmittel gelöst werden.

23. Verfahren nach Anspruch 21 oder 22, wobei der Dampfdruck des organischen Lösungsmittels bei 20 °C mehr als 0,025 bar, insbesondere mehr als 0,027 bar, vorzugsweise mehr als 0,03 bar, beträgt.

24. Verfahren nach einem der Ansprüche 21 bis 23, wobei das organische Lösungsmittel Methanol, Ethanol, Propanol, Isopropylalkohol, Butanon, Aceton, Ethylether, Benzol oder eine Mischung von mindestens zwei dieser Lösungsmittel ist.

25. Verfahren nach einem der Ansprüche 21 bis 24, wobei die Konzentration des organischen Lösungsmittels in der Markierungslösung auf einen Wert größer als 50 % (v/v), bevorzugt größer als 80 % (v/v), insbesondere größer als 90 % (v/v), eingestellt wird.

26. Verfahren nach einem der Ansprüche 21 bis 25, wobei die Konzentration von Wasser in der Markierungslösung auf einen Wert kleiner als 20 % (v/v), bevorzugt kleiner als 10 % (v/v), insbesondere kleiner als 5 % (v/v), eingestellt wird.

27. Verfahren nach einem der Ansprüche 21 bis 26, wobei die Konzentration des Hilfsstoffs in der Markierungslösung auf einen Wert zwischen 0,1 % (w/v) und 10 % (w/v), insbesondere zwischen 0,5 % (w/v) und 5 % (w/v), vorzugsweise zwischen 0,7 % (w/v) und 3 % (w/v), eingestellt wird.

28. Verfahren nach einem der Ansprüche 21 bis 27, wobei der Markierungslösung, dem wässrigen Lösungsmittel oder dem organischen Lösungsmittel ein, insbesondere fluoreszierender, Farbstoff zugesetzt wird.

29. Verfahren nach einem der Ansprüche 21 bis 28, wobei die ersten Nukleinsäuren einzelsträngig vorliegen.

30. Verfahren nach einem der Anspruche 21 bis 29, wobei der Markierungslösung, dem wässrigen Lösungsmittel oder dem organischen Lösungsmittel visköse Polymere zur Einstellung der Viskosität der Markierungslösung und/oder geladene Moleküle, insbesondere Tetrabutylammoniumbromid, zur Einstellung der Leitfähigkeit der Markierungslösung zugesetzt werden.

31. Verfahren nach einem der Ansprüche 21 bis 30, wobei die ersten Nukleinsäuren eine Länge von 5 bis 300 Nukleotiden, insbesondere 10 bis 100 Nukleotiden, vorzugsweise 15 bis 25 Nukleotiden, aufweisen.

32. Verfahren nach einem der Ansprüche 21 bis 31, wobei in dem wässrigen Lösungsmittel neben den ersten Nukleinsäuren, insbesondere dieselbe oder nahezu dieselbe Länge wie die ersten Nukleinsäuren aufweisende, weitere Nukleinsäuren gelöst werden oder neben den ersten Nukleinsäuren weitere Nukleinsäuren in dem wässrigen Lösungsmittel bereitgestellt werden.

33. Verfahren nach Anspruch 32, wobei das Mengenverhältnis zwischen den ersten und den weiteren Nukleinsäuren höchstens 1:10, bevorzugt höchstens 1:20, insbesondere höchstens 1:100, beträgt.

34. Verfahren nach einem der Ansprüche 21 bis 33, wobei die Konzentration der ersten oder der ersten und der weiteren Nukleinsäuren in der Markierungslösung auf einen Wert eingestellt wird, der höher als 0,1 % (w/v), insbesondere höher als 1 % (w/v), vorzugsweise höher als 2 % (w/v), ist.

35. Verfahren nach einem der Ansprüche 21 bis 34, wobei der Hilfsstoff ein Detergens ist.

36. Verfahren nach einem der Ansprüche 21 bis 35, wobei das Detergens ein kationisches Detergens ist.

37. Verfahren nach Anspruch 36, wobei das kationische Detergens Hexadecyltrimethylammoniumbromid oder Dodecyltrimethylammoniumbromid ist.

38. Verfahren nach einem der Ansprüche 21 bis 37, wobei der Hilfsstoff ein monomeres oder polymeres Amin, vorzugsweise Spermidin oder Polylysin, ist.

39. Verfahren nach einem der Ansprüche 32 bis 38, wobei die ersten und weiteren Nukleinsäuren in dem organischen Lösungsmittel in einer Konzentration gelöst werden, in welcher sie ohne den Hilfsstoff in dem organischen Lösungsmittel nicht vollständig löslich wären.

40. Verwendung einer Markierungslösung, die als Komponenten (i) mindestens ein organisches Lösungsmittel mit einem bei 20 °C höheren Dampfdruck als Wasser und einem Wassergehalt von weniger als 50% (v/v), (ii) vorgegebene erste Nukleinsäuren und (iii) einen die ersten Nukleinsäuren komplexierenden, organischen Hilfsstoff enthält, zum Markieren einer Oberfläche eines Objekts mittels eines Druckers.

41. Verwendung nach Anspruch 40, wobei der Drucker ein Tintenstrahldrucker ist.

42. Verwendung nach Anspruch 40 oder 41, wobei die Oberfläche glatt ist.

43. Verwendung nach einem der Ansprüche 40 bis 42, wobei die Oberfläche aus einem hydrophoben Kunststoff besteht.

44. Verwendung nach Anspruch 43, wobei der Kunststoff Polyethylen oder Polypropylen ist.

45. Verwendung nach einem der Ansprüche 4 0 bis 44, wobei eine Markierung **dadurch** gebildet wird, dass die Markierungslösung auf definierte Flächen und eine andere Lösung auf weitere Flächen aufgebracht wird, wobei die andere Lösung wie die Markierungslösung zusammengesetzt ist, jedoch keine ersten Nukleinsäuren enthält.

46. Verwendung nach Anspruch 45, wobei die andere Lösung zweite Nukleinsäuren enthält.

47. Verfahren zum Markieren einer Oberfläche eines Objekts, bei dem man eine Markierungslösung nach einem der Ansprüche 1 bis 19 mittels eines Druckers auf die Oberfläche aufträgt.

48. Druckkopf eines Tintenstrahldruckers, wobei der Druckkopf die Markierungslösung gemäß einem der Ansprüche 1 bis 20 in einem Vorratsbehälter oder in einem Kanal enthält.

49. Tintenstrahldrucker, der einen Druckkopf gemäß Anspruch 48 enthält.

## Claims

1. Marker solution for application with an inkjet printer, comprising as components (i) at least one organic solvent with a vapour pressure that is higher at 20°C than that of water and a water content of less than 50% (v/v), (ii) predefined first synthetically produced nucleic acids and (iii) an organic additive that complexes the first nucleic acids.

2. Marker solution according to claim 1, wherein the marker solution has a higher vapour pressure at 20°C than water.

3. Marker solution according to any of the previous claims, wherein the vapour pressure of the organic solvent or the marker solution at 20°C is more than 0.025 bar, particularly more than 0.027 bar, preferably more than 0.03 bar.

4. Marker solution according to any of the previous claims, wherein the organic solvent is methanol, ethanol, propanol, isopropyl alcohol, butanone, acetone, ethyl ether, benzol, or a mixture of at least two of these solvents.

5. Marker solution according to any of the previous claims, wherein the concentration of the organic solvent in the marker solution is higher than 50% (v/v), preferably higher than 80% (v/v), and more preferably higher than 90% (v/v) .

6. Marker solution according to any of the previous claims, wherein the concentration of water in the marker solution is less than 20% (v/v), preferably less than 10% (v/v), particularly less than 5% (v/v).

7. Marker solution according to any of the previous claims, wherein the marker solution further comprises a dye, preferably a luminescent dye, and more preferably fluorescent dye.

8. Marker solution according to any of the previous claims, wherein the first nucleic acids are single-stranded.

9. Marker solution according to any of the previous claims, comprising viscous polymers for the adjustment of the viscosity of the marker solution and/or charged molecules, for the adjustment of the conductivity of the marker solution, particularly tetrabutylammoniumbromide.

10. Marker solution according to any of the previous claims, wherein the first nucleic acids have a length of between 5 and 300 nucleotides, particularly 10 to 100 nucleotides, preferably 15 to 25 nucleotides.

11. Marker solution according to any of the previous claims, comprising further nucleic acids having the same or nearly the same length as the first nucleic acids.

12. Marker solution according to claim 11, wherein the quantitative ratio between the first and the further nucleic acids is not exceeding 1:10, preferably not exceeding 1:20 and particularly not exceeding 1:100.

13. Marker solution according to any of the previous claims, wherein the concentration of the first nucleic acids or the first and the further nucleic acids in the marker solution is higher than 0.1% (w/v), particularly higher than 1% (w/v) and preferably higher than 2% (w/v).

14. Marker solution according to any of the previous claims, wherein the additive complexing the first nucleic acids is a detergent with one or more positive charges.

15. Marker solution according to claim 14, wherein the concentration of the detergent in the marker solution is between 0.1% (w/v) and 10% (w/v), particularly between 0.5% % (w/v) and 5% (w/v), preferably between 0.7 % (w/v) and 3% (w/v).

16. Marker solution according to any of the previous claims, wherein the additive complexing the first nucleic acids is a monomeric or polymeric amine, such as spermidine or poly-lysine.

17. Marker solution according to any of the previous claims, wherein the additive is a cationic detergent.

18. Marker solution according to claim 17, wherein the cationic detergent is hexadecyltrimethyl-ammoniumbromide or dodecyltrimethyl-ammoniumbromide.

19. Marker solution according to any of the previous claims, wherein the first or the further nucleic acids form complexes with the additive which are insoluble in aqueous solution.

20. Marker solution according to any one of claims 11 to 19, wherein the solubility of the first and the further nucleic acids in the marker solution is a factor 5, preferably a factor 10, more preferably a factor 100 higher than in an identical solution without the additive.

21. Method for producing the marker solution according to any one of claims 1 to 20, comprising the following steps:
a) solving synthetically produced first nucleic acids in an aqueous solvent or providing synthetically produced first nucleic acids in an aqueous solvent,
b) contacting the first nucleic acids with an organic additive that complexes the first nucleic acids, wherein the first nucleic acids and the additive form complexes,
c) mixing the complexes with an organic solvent that has a higher vapour pressure at 20 °C than water.

22. Method according to claim 21, wherein complexes that precipitate in step b) are separated from the aqueous solvent and dissolved in the organic solvent in step c).

23. Method according to claim 21 or 22, wherein the vapour pressure of the organic solvent at 20 °C is higher than 0.025 bar, particularly higher than 0.027 bar, and preferably higher than 0.03 bar.

24. Method according to any one of claims 21 to 23, wherein the organic solvent is methanol, ethanol, propanol, isopropyl alcohol, butanone, acetone, ethyl ether, benzol, or a mixture of at least two of these solvents.

25. Method according to any one of claims 21 to 24, wherein the concentration of the organic solvent in the marker solution is adjusted to a value higher than 50% (v/v), preferably higher than 80% (v/v), particularly higher than 90% (v/v).

26. Method according to any one of claims 21 to 25, wherein the concentration of the water in the marker solution is adjusted to a value less than 20% (v/v), preferably less than 10% (v/v), particularly less than 5% (v/v).

27. Method according to any one of claims 21 to 26, wherein the concentration of the additive in the marker solution is adjusted to a value between 0.1% (w/v) and 10% (w/v), particularly between 0.5% (w/v) and 5% (w/v), preferably between 0.7% (w/v) and 3% (w/v) .

28. Method according to any one of claims 21 to 27, wherein a dye, particularly a fluorescent dye, is added to the marker solution, the aqueous solvent or the organic solvent.

29. Method according to any one of claims 21 to 28, wherein the first nucleic acids are single-stranded.

30. Method according to any one of claims 21 to 29, wherein viscous polymers for the adjustment of the viscosity of the marker solution and/or charged molecules, particularly tetrabutylammoniumbromide, for the adjustment of the conductivity of the marker solution are added to the marker solution, the aqueous solvent or the organic solvent.

31. Method according to any one of claims 21 to 30, wherein the first nucleic acids have a length of between 5 and 300 nucleotides, particularly 10 to 100 nucleotides, preferably 15 to 25 nucleotides.

32. Method according to any one of claims 21 to 31, wherein further nucleic acids having the same or nearly the same length as the first nucleic acids are dissolved in the aqueous solvent in addition to the first nucleic acids or wherein further nucleic acids are provided in the aqueous solvent in addition to the first nucleic acids.

33. Method according to claim 32, wherein the quantitative ratio between the first and the further nucleic acids is not exceeding 1:10, preferably not exceeding 1:20 and particularly not exceeding 1:100.

34. Method according to any one of claims 21 to 33, wherein the concentration of the first nucleic acids or the first and the further nucleic acids in the marker solution is adjusted to a value which is higher than 0.1% (w/v), particularly higher than 1% (w/v) and preferably higher than 2% (w/v).

35. Method according to any one of claims 21 to 34, wherein the additive is a detergent.

36. Method according to any one of claims 21 to 35, wherein the detergent is a cationic detergent.

37. Method according to claim 36, wherein the cationic detergent is hexadecyltrimethyl-ammoniumbromide or dodecyltrimethyl-ammoniumbromide.

38. Method according to any one of claims 21 to 37, wherein the additive is a monomeric or polymeric amine, preferably spermidine or poly-lysine.

39. Method according to any one of claims 32 to 38, wherein the first and the further nucleic acids are dissolved in the organic solvent in a concentration at which they would not be completely soluble in the organic solvent in the absence of the additive.

40. Use of a marker solution that comprises as components (i) at least one organic solvent with a vapour pressure that is higher at 20°C than that of water and a water content of less than 50% (v/v), (ii) predefined first nucleic acids and (iii) an organic additive that complexes the first nucleic acids, for marking the surface of an object by means of a printer.

41. Use according to claim 40, wherein the printer is an inkjet printer.

42. Use according to claim 40 or 41, wherein the surface is smooth.

43. Use according to any one of claims 40 to 42, wherein the surface consists of a hydrophobic plastic.

44. Use according to claim 43, wherein the plastic is polyethylene or polypropylene.

45. Use according to any one of claims 40 to 44, wherein a mark is formed by applying the marker solution to defined areas and applying another solution to further areas, wherein the other solution is composed like the marker solution, but does not contain first nucleic acids.

46. Use according to claim 45, wherein the other solution comprises second nucleic acids.

47. Method for marking a surface of an object, comprising applying a marker solution according to any one of claims 1 to 19 to the surface by means of a printer.

48. Print head of an inkjet printer, wherein said print head contains the marker solution according to any one of claims 1 to 20 in a storage container or in a channel.

49. Inkjet printer, comprising a print head according to claim 48.

## Revendications

1. Solution de marquage à appliquer au moyen d'une imprimante à jet d'encre, qui comprend comme composants (i) au moins un solvant organique présentant une pression de vapeur à 20° C supérieure à celle de l'eau et une teneur en eau inférieure à 50 % (v/v), (ii) des acides nucléiques prédéterminés produits synthétiquement et (iii) un adjuvant organique complexant lesdits acides nucléiques.

2. Solution de marquage selon la revendication 1, présentant à 20° C une pression de vapeur supérieure à celle de l'eau.

3. Solution de marquage selon l'une des revendications précédentes, dans laquelle la pression de vapeur à 20° C du solvant organique ou de la solution de marquage est supérieure à 0,025 bars, en particulier supérieure à 0,027 bars, de préférence supérieure à 0,03 bars.

4. Solution de marquage selon l'une des revendications précédentes, dans laquelle le solvant organique est le méthanol, l'éthanol, le propanol, l'isopropanol, le butanone, l'acétone, l'éthyléther, le benzène ou un mélange d'au moins deux de ces solvants.

5. Solution de marquage selon l'une des revendications précédentes, dans laquelle la concentration du solvant organique dans la solution de marquage est supérieure à 50% (v/v), de préférence supérieure à 80% (v/v), de manière encore plus préférée supérieure à 90% (v/v).

6. Solution de marquage selon l'une des revendications précédentes, dans laquelle la concentration d'eau dans la solution de marquage est inférieure à 20 % (v/v), de préférence inférieure à 10% (v/v), en particulier, inférieure à 5% (v/v).

7. Solution de marquage selon l'une des revendications précédentes, comprenant en outre un colorant, de préférence, luminescent, de minière particulièrement préférée, fluorescent.

8. Solution de marquage selon l'une des revendications précédentes, dans laquelle les acides nucléiques prédéterminés sont à simple brin.

9. Solution de marquage selon l'une des revendications précédentes, dans laquelle sont contenus des polymères visqueux pour ajuster la viscosité de la solution de marquage et/ou des molécules chargées, en particulier le bromure de tétrabutylammonium, pour l'ajustement de la conductibilité de la solution de marquage.

10. Solution de marquage selon l'une des revendications précédentes, dans laquelle les acides nucléiques prédéterminés ont une longueur de 5 à 300 nucleotides, en particulier de 10 à 100 nucléotides, de préférence, de 15 à 25 nucleotides.

11. Solution de marquage selon l'une des revendications précédentes, comprenant d'autres acides nucléiques, de préférence de même longueur ou pratiquement de même longueur que les premiers acides nucléiques prédéterminés.

12. Solution de marquage selon la revendication 11, dans laquelle le rapport de quantités entre les premiers et les autres acides nucléiques est au plus de 1:10, de préférence au plus de 1:20, de manière encore plus préférée au plus de 1:100.

13. Solution de marquage selon l'une des revendications précédentes, dans laquelle la concentration des premiers ou des premiers et des autres acides nucléiques dans la solution de marquage est supérieure à 0,1% (p/v), en particulier supérieure à 1 % (p/v), de préférence supérieure à 2 % (p/v).

14. Solution de marquage selon l'une des revendications precédentes, dans laquelle l'adjuvant complexant les acides nucléiques prédéterminés est un détergent portant une ou plusieurs charges positives.

15. Solution de marquage selon la revendication 14, dans laquelle la concentration de détergent dans la solution de marquage se situe entre 0,1% (p/v) et 10 % (p/v), en particulier entre 0,5% (p/v) et 5 % (p/v), de préférence entre 0,7% (p/v) et 3% (p/v) .

16. Solution de marquage selon l'une des revendications précédentes, dans laquelle l'adjuvant complexant les acides nucléiques prédéterminés est une amine mononérique ou polymérique telle que par exemple, la spermidine ou la polylysine.

17. Solution de marquage selon l'une des revendications précédente, dans laquelle l'adjuvant est un détergent cationique.

18. Solution de marquage selon la revendication 17, dans laquelle le détergent cationique est la bromure d'hexadécyltriméthylammonium ou le bromure de dodécyltriméthylammonium.

19. Solution de marquage selon l'une des revendications précédentes, dans laquelle les premiers ou les autres acides nucléiques forment des complexes avec l'adjuvant, qui sont insolubles dans une solution aqueuse.

20. Solution de marquage selon l'une des revendications 11 a 19, dans laquelle la solubilité des premiers et des autres acides nucléiques dans la solution de marquage est d'un facteur 5, de préférence d'un facteur 10, de manière particulièrement préférée, d'un facteur 100 par rapport à la solubilité dans une solution identique sans adjuvant.

21. Procédé de production de la solution de marquage selon l'une des revendications 1 à 20, comportant les étapes suivantes:
a) dissolution des acides nucléiques prédéterminés produits synthétiquement dans un solvant aqueux ou mise à disposition de acides nucléiques prédéterminés produits synthétiquement dans un solvant aqueux,
b) mise en contact des acides nucléiques prédéterminés avec un adjuvant organique complexant lesdits acides nucléiques prédéterminés, des complexes se formant à partir desdits acides nucléiques prédéterminés et de l'adjuvant,
c) mélange des complexes avec un solvant organique qui présente à 20° C, une pression de vapeur supérieure à celle de l'eau.

22. Procédé selon la revendication 21, dans lequel à l'étape selon le point b), des complexes formés à partir du solvant aqueux sont séparés et à l'étape c), sont dissous dans le solvant organique.

23. Procédé selon la revendication 21 ou 22, dans lequel la pression de vapeur du solvant organique à 20° C est supérieure à 0,025 bars, en particulier, supérieure à 0,027 bars, de préférence, supérieure à 0,03 bars.

24. Procédé selon l'une des revendications 21 à 23, dans lequel le solvant organique est le méthanol, l'éthanol, le propanol, l'isopropanol, le butanone, l'acétone, l'éthyléther, le benzène ou un mélange d'au moins deux de ces solvants.

25. Procédé selon l'une des revendications 21 à 24, dans lequel la concentration du solvant organique dans la solution de marquage est ajustée à une valeur supérieure à 50 % (v/v), de préférence supérieure à 80 % (v/v), de manière encore plus préférée supérieure à 90% (v/v).

26. Procédé selon l'une des revendications 21 a 25, dans lequel la concentration d'eau dans la solution de marquage est ajustée à une valeur inférieure à 20% (v/v), de préférence inférieure à 10% (v/v), de manière encore plus préférée, inférieure à 5 % (v/v).

27. Procédé selon l'une des revendications 21 à 26, dans lequel la concentration d'adjuvant dans la solution de marquage est ajustée à une valeur entre 0,1% (p/v) et 10% % (p/v), en particulier entre 0,5 % (p/v) et 5% (v/v), de préférence entre 0,7 % (p/v) et 3% (p/v).

28. Procédé selon l'une des revendications 21 à 27, dans lequel à la solution de marquage, au solvant aqueux ou au solvant organique, on ajoute un colorant, de préférence fluorescent.

29. Procédé selon l'une des revendications 21 à 28, dans lequel les acides nucléiques prédéterminés sont à simple brin.

30. Procédé selon l'une des revendications 21 a 29, dans lequel, à la solution de marquage, au solvant aqueux ou au solvant organique, on ajoute des polymères visqueux pour ajuster la viscosité de la solution de marquage et/ou des molécules chargées, en particulier le bromure de tetrabutylammonium, pour l'ajustement de la conductibilité de la solution de marquage.

31. Procédé selon l'une des revendications 21 à 30, dans lequel les acides nucléiques prédéterminés ont une longueur de 5 à 300 nucléotides, en particulier de 10 à 100 nucléotides, de préférence, de 15 à 25 nucléotides.

32. Procédé selon l'une des revendications 21 à 31, dans lequel sont dissous dans le solvant aqueux, outre les premiers acides nucléiques prédéterminés, d'autres acides nucléiques en particulier de même longueur ou pratiquement de même longueur que les premiers acides nucléiques ou dans lequel, outre les premiers acides nucléiques prédéterminés sont mis à disposition d'autres acides nucléiques dans le solvant aqueux.

33. Procédé selon la revendications 32, dans lequel le rapport de quantités entre les premiers et les autres acides nucléiques est au plus de 1:10, de préférence au plus de 1:20, en particulier au plus de 1:100.

34. Procédé selon l'une des revendications 21 à 33, dans lequel la concentration des premiers ou des premiers et des autres acides nucléiques dans la solution de marquage est ajustée à une valeur supérieure à 0,1 % (p/v), en particulier, supérieure à 1% (p/v), de préférence supérieure à 2% (p/v).

35. Procédé selon l'une des revendications 21 à 34, dans lequel l'adjuvant est un détergent.

36. Procédé selon l'une des revendications 21 à 35, dans lequel le détergent est un détergent cationique.

37. Procédé selon la revendication 36, dans lequel le détergent cationique est le bromure d'hexadécyltriméthylammonium ou le bromure de dodécyltriméthylammonium.

38. Procédé selon l'une des revendications 21 à 37, dans lequel l'adjuvant est une amine monomérique ou polymérique, de préférence, la spermidine ou la polylysine.

39. Procédé selon l'une des revendications 32 à 38, dans lequel les premiers et les autres acides nucléiques sont dissous dans le solvant organique en une concentration dans laquelle ils ne sont pas complétement solubles dans le solvant organique sans l'adjuvant.

40. Utilisation d'une solution de marquage qui contient comme composants (i) au moins un solvant organique présentant une pression de vapeur à 20° C supérieure à celle de l'eau et une teneur en eau inférieure à 50% (v/v), (ii) des premiers acides nucléiques prédéterminés et (iii) un adjuvant organique complexant les premiers acides nucléiques, pour le marquage d'une surface d'un objet au moyen d'une imprimante.

41. Utilisation selon la revendication 40, dans laquelle l'imprimante est une imprimante à jet d'encre.

42. Utilisation selon la revendication 40 ou 41, dans laquelle la surface est lisse.

43. Utilisation selon l'une des revendications 40 à 42, dans laquelle la surface est constituée d'un plastique hydrophobe.

44. Utilisation selon la revendication 43, dans laquelle le plastique est le polyéthylène ou le polypropylène.

45. Utilisation selon l'une des revendications 40 à 44, dans laquelle un marquage est formé par l'application de la solution de marquage sur des surfaces définies et par l'application d'une autre solution sur d'autres surfaces, l'autre solution étant composée comme la solution de marquage mais ne contenant toutefois pas de premiers acides nucléiques.

46. Utilisation selon la revendication 45, dans laquelle l'autre solution contient des deuxièmes acides nucléiques.

47. Procédé de marquage d'une surface d'un objet, dans lequel on applique une solution de marquage selon l'une des revendications 1 à 19 au moyen d'une imprimante sur la surface.

48. Tête d'impression d'une imprimante à jet d'encre, la tête d'impression contenant la solution de marquage selon l'une des revendications 1 à 20, dans un réservoir ou dans un canal.

49. Imprimante à jet d'encre qui contient une tête d'impression selon la revendication 48.
